# EUROPEAN PATENT APPLICATION

(11) **EP 2 606 880 A1**
(43) Date of publication of application: **26.06.2013**
(21) Application number: 11195532.4
(22) Date of filing: 23.12.2011
(51) Int. Cl.: A61K 9/50

(54) **Coating process with aqueous latex coating**

(71) Applicant: LEK Pharmaceuticals d.d., 1526 Ljubljana (SI)
(72) Inventor: Staric, Rok, 1526 Ljubljana (SI); Cesar, Sara, 1526 Ljubljana (SI)
(74) Representative: Kunic Tesovic, Barbara

(57) **Abstract**

The present invention refers to a process for coating a substrate with an aqueous coating dispersion, as well as to a production batch comprising coated substrate. Further, the present invention refers to a coated substrate comprising acid-labile API and coalescent coating.

## Description

### Field of the invention

The present invention belongs to the field of pharmaceutical industry and relates to a process for the manufacture of a coated substrate by using an aqueous latex or pseudolatex dispersion. Further, the present invention refers to a production batch comprising said coated substrates.

### Description of the background art

Coating processes, such as fluid bed coating, spouted bed coating or drum coating, are widely used for preparing pharmaceutical dosage forms. In general, such coatings are applied in order to modify the surface characteristics of the substrates to be coated. For instance, a coating provides for controlled-release, taste masking, and/or stabilization of an active pharmaceutical ingredient (API) in a dosage form. Substrates which can be coated in coating processes are for example beads, pellets, and spheroids. In general, in coating processes, as coating fluids aqueous systems such as aqueous coating dispersions are used, or non-aqueous systems such as organic coating solutions. An example of aqueous coating dispersions are aqueous latex or pseudolatex dispersions.

Coating processes can for example be carried out in drum coating systems, fluid-bed systems (e.g. a Wurster coating) or spouted bed systems by spraying the coating fluid on a substrate and drying said coating fluid on the substrate to form the desired coating. In general, coating devices are available and process conditions for the coating process are known to a person skilled in the art.
However, choosing process parameters of the spray-drying process can be critical for the quality of the resulting coating.

A process of spray-coating tablets with an aqueous latex dispersion is for instance disclosed in US 5,047,258. Therein, an inlet air having a low humidity as expressed by a low dew point of below 10°C is used. As further described in said document, by using such a low humidity inlet air, and by maintaining the dew point of the process or inlet air below about 10°C, the presence of a glidant is not necessary for the successful application of an Eudragit ® L30D enteric coat. The Eudragit ® L30D enteric coating dispersion used in US 5,047,258 is diluted with water.

US 7,829,148 B2 describes a coating process where the humidity in the coating chamber is influenced by either addition of water to the coating chamber, by diluting the coating formulation or by humidification of inlet air. The coating process includes a heat treatment of the coated substrate above the film forming temperature of the coating at low humidity in order to improve the quality of the coating.

There is still a need and thus an object for improved coating processes.

### Summary of the invention

The present invention provides the following aspects, subject-matters and preferred embodiments, which respectively taken alone or in combination, contribute to solving the object of the present invention:
(1) A process for the manufacture of a coated substrate having a coalescent coating, the process comprising the following steps:
   a) providing an aqueous latex or pseudolatex dispersion with a solid content of from 20% to 40% by weight (w/w),
   b) coating the substrate with the aqueous latex or pseudolatex dispersion of step a),
      wherein
      the step of coating the substrate comprises a coating phase that is carried out at a defined relative air humidity which is measured or determined inside the coating chamber or in the outlet air from the coating chamber where the process air exits the coating chamber, with said defined relative air humidity being at least 35% during a time period of at least 60%, preferably a time period of at least 70%, further preferred of at least 80% or 100%, of the duration of the coating phase, and at a product temperature of equal to or more than 30°C,
      wherein the relative air humidity is adjusted by setting a spray rate of the aqueous latex or pseudolatex dispersion,
      and
   c) obtaining a coated substrate having coalescent coating.

Herein, the term "in the outlet air from the coating chamber" defines the outlet air humidity in the process air which exits the coating chamber. The process air that exits the coating chamber is also referred to herein as "outlet air". During measuring the humidity of the outlet air, a mixing of outlet air and ambient air surrounding the coating device has to be avoided.

Further in the present specification, the term "step of coating the substrate" may sometimes also be referred to as "coating step". Typically, this coating step, which preferably takes place in a coating chamber of a coating apparatus, may comprise various process phases, such as a pre-heating phase, a coating phase, a drying phase (which can take place simultaneously, or being overlapping with, the coating phase), and/or a cool-down phase (which can also take place simultaneously, or being overlapping with, the drying phase).
In the pre-heating phase, the substrate to be coated and/or the coating chamber can be preheated to a defined temperature. It is possible to pre-heat the coating apparatus or coating chamber prior to introducing the substrate to be coated into the coating apparatus/coating chamber. It is also possible to introduce the substrate to be coated into the coating apparatus/coating chamber, and then pre-heat said apparatus/chamber to the desired temperature.

Within the meaning of the present invention, the term "coating phase" denotes the phase that starts with the introduction of the coating dispersion, i.e. the aqueous latex or pseudolatex dispersion, into the coating chamber, and ends once no coating dispersion is introduced into the coating chamber any more. During the coating phase, the introduced coating dispersion is applied onto the substrate being present in the coating chamber.

Further, a coating step may comprise a drying phase and/or a cool-down phase of the coated substrate. During the drying phase, the coating dispersion applied onto the substrate to be coated dries without adding further aqueous latex or pseudolatex dispersion, the solids being present in the coating dispersion remaining on the substrate and thereby forming the coating.

As it is known to a person skilled in the art, the relative humidity is a measure of the amount of water vapour in the air (at a specific temperature) compared to the maximum amount of water vapour air could hold at that temperature, and is given as a percentage value. Further, absolute humidity is the actual measurable amount of water vapour in a parcel of air.

Within the meaning of the present invention, the term "aqueous latex or pseudolatex dispersion" refers to a dispersion of water insoluble film forming materials which upon drying or at least substantive removal of water are capable of coalescing to form a coating on a substrate.
Latexes can e.g. be prepared by polymerization of a monomer or monomer blend which is usually emulsified in an aqueous medium of anionic or non-ionic surfactants.
Pseudolatexes can be prepared by emulsifying preformed polymers. This can e.g. be done by dissolving a polymer, e.g. ethylcellulose or other cellulose derivatives or (meth)acrylate copolymers, in a suitable organic solvent, combining said solution with water to provide an emulsion, whereupon removal of the organic solvent by distillation a pseudolatex dispersion in water is obtained. When preparing pseudolatex or latex dispersions, it may be useful to use emulsifiers.

By maintaining the relative air humidity inside the coating chamber in the range as indicated herein by setting a spray rate of the aqueous latex or pseudolatex dispersion, it is possible to obtain coatings that are improved, e.g. in terms of uniformity of the coating. Further, when applying the process according to the present invention, the obtained coating is essentially free of damages or even entirely free of damages, so that unwanted substantial early drug release under acidic conditions is effectively avoided. Furthermore, as the obtained coating is by itself essentially free, or even entirely free, of damages, it may be possible that there is no need for compensating damages that are otherwise (e.g. if prior art method is used) present in the coating by applying a thicker coating. Therefore, the preferred possibility to make the coating thinner while still having essential or even total freeness of defects brings about substantial advantages, e.g. may reduce material needs, assist to reduce overall size, further reduce costs and safe time. Moreover, the coated substrate can exhibit improved properties with regard to the dissolution of the API being present in the coated substrate, e.g. with regard to a constant or, if desired, a controlled release of the API.
(2) The process according to item (1), wherein during a time period of at least 60% of the duration of the coating phase the relative air humidity inside the coating chamber is maintained essentially constant at a value of at least 35%, and/or is increased from a value of at least 35%.

Within the meaning of the present invention, "maintaining" a certain level of relative air humidity means that a drop in relative air humidity is compensated by accordingly adapting the spray rate.

In other words, in one embodiment of the process according to the present invention, the relative air humidity inside the coating chamber has a certain value, with this value being at least 35%, and this value is maintained essentially constant during a time period of at least 60% of the coating phase. Within the meaning of the present invention, the term "maintained essentially constant" denotes that during the coating phase the relative air humidity inside the coating chamber does not deviate by more than +/- 8%, preferably not more than +/- 5% more preferably not more than +/- 3% of the value.

However, it is also possible that during at least 60% of the coating phase the relative air humidity inside the coating chamber, being at least 35%, is further increased from said value. In one further embodiment, the relative air humidity inside the coating chamber can be increased until a certain maximum relative air humidity is reached.

Maintaining the relative air humidity inside the coating chamber essentially constant at a value of at least 35%, and/or by increasing the relative air humidity inside the coating chamber from a value of at least 35%, represents a smooth coating process that further contributes to a uniform coalescent coating process, while at the same time essentially avoiding the occurrence of damages such as fissure, breakings and/or cracks in the coating.
(3) The process according to items (1) or (2), wherein in step b) the relative air humidity in the coating chamber is maintained and/or is increased during a time period of at least 65%, 70% or 75% of the coating phase, more preferably during at least 80% or 85%, even more preferred during at least 90% or 95%.
(4) The process according to any of the preceding items, wherein in step b) the relative air humidity is more than 35%, preferably more than 37%, further preferred more than 39%, further preferred 41% or more, even further preferred 43% or more.
(5) The process according to any of the preceding items, wherein the aqueous latex or pseudolatex dispersion has a solid content from 27% to 40% by weight, preferably from 27% to 35% by weight, and more preferably from 28% to 34% by weight.

In an even more preferred embodiment, the aqueous latex or pseudolatex dispersion has a solid content of about 32% by weight.

Within the meaning of the present invention, the term "about" used herein has its typical meaning in the respective context. For example, it means tolerance ranges of +/-10%, +/-7%, +/-5%, +/-4%, +/-3%, +/-2%, or +/-1% of the indicated value.

The use of an aqueous latex dispersion exhibiting the above-indicated solid content can even more contribute to arriving at a coalescent coating.
(6) The process according to any of the preceding items, wherein maintaining the relative air humidity essentially constant, and/or increasing the relative air humidity, is performed by continuously, gradually or intermittently, increasing the spray rate of the aqueous latex or pseudolatex dispersion.
(7) The process according to any of the preceding items, wherein coating the substrate in step b) is carried out by applying a spray coating process.
(8) The process according to any of the preceding items, wherein the product temperature of step b) is the product bed temperature.
(9) The process according to any of the preceding items, wherein after step b) no heat treatment step comprising applying a temperature above a minimum film forming temperature of the coating is carried out.

In a preferred embodiment according to the present invention, no heat treatment step comprising applying a temperature above a minimum film forming temperature of the coating is carried out.

Within the meaning of the present invention, the term "film forming temperature" denotes the lowest temperature at which a latex or pseudolatex dispersion will uniformly coalesce when laid on a substrate e.g. as a thin film.

By maintaining the relative air humidity inside the coating chamber in the range as indicated herein by setting a spray rate of the aqueous latex or pseudolatex dispersion, it is possible to avoid, if desired, a (further) step of heat treatment after step b) comprising applying a temperature above a minimum film forming temperature of the coating. This can further contribute to improving the process, e.g. with regard to the process time.

Moreover, the avoidance of a (further) step of heat treatment (after step b), or, preferably, during the whole process), i.e. avoiding a temperature above a minimum film forming temperature, can provide advantages if heat labile APIs are used, e.g. with regard to reducing the degradation of the API that is present in the substrate to be coated.
(10) The process according to any of the preceding items, wherein in step b) the relative air humidity is maintained essentially constant, or increased, without providing further amounts of water in addition to the water that is already present in the aqueous latex or pseudolatex dispersion, and/or without spraying water into the coating chamber, and/or without addition of water to the process air.

In one embodiment, an aqueous coating dispersion is used in the process according to the present invention. A commercially available aqueous coating dispersion can be suitably used. An aqueous dispersion, once suitably prepared or chosen from commercially available ones, is preferably not (further) diluted by adding additional amounts of water (e.g. to such commercially available dispersion) prior or during the introduction of the coating dispersion. Generally suitable contents of water lie in the range of 50%-80% (w/w), preferably 60%-70% (w/w) (such as 60% (w/w) or 70% (w/w)).

Examples of commercially available aqueous coating dispersions are for instance the commercially available members of the Eudragit ® aqueous coating dispersion series of Evonik Industries, such as Eudragit ® L 30 D, FS 30 D, RL 30 D, RS 30 D, NE 30 D, NE 40 D, or NM 30 D, preferably Eudragit ® L 30 D, FS 30 D, RL 30 D, RS 30 D, NE 30 D, or NM 30 D. The amount of water in this commercially available coating dispersions is 70% (w/w), or 60% (w/w), respectively (in the case of Eudragit ® NE 40).

In a further embodiment, if a substance such as a powder for mixing coating dispersions is commercially available, this substance is used for preparing aqueous coating dispersions having a solid content as described herein, and/or as recommended in the corresponding leaflet. It is not (further) diluted by adding additional amounts of water. An example of a commercially available substance that can be used for preparing an aqueous coating composition is the Aquacoat ® ECD series available for instance from FMC BioPolymer.
(11) The process according to any of the preceding items, wherein after step c) a further coating is applied onto the coated substrate.
(12) The process according to item (11), wherein said further coating includes colorant/s and/or flavour/s.
(13) The process according to any of the preceding items, wherein the substrate is selected from the group consisting of pellets, tablets, granules, grains, soft capsules, hard capsules, powders, beads, films, or spheroids.
(14) The process according to any of the preceding items, wherein the substrate comprises pharmaceutically active ingredient (API), preferably acid-labile API.
(15) The process according to item (14), wherein the acid-labile API is selected from the group consisting of lansoprazole, omeprazole, esomeprazole, pantoprazole, rabeprazole, leminoprazole, hydroxyomeprazole, pariprazole, perprazole, tenatoprazole, and pharmaceutically acceptable salts thereof, preferably from the group consisting of lansoprazole, omeprazole, esomeprazole, pantoprazole, rabeprazole, and pharmaceutically acceptable salts thereof, and more preferably the acid-labile API is omeprazole or a pharmaceutically acceptable salt thereof.
(16) The process according to any of the preceding items, wherein the aqueous latex or pseudolatex dispersion comprises one or more polymers selected from the group consisting of ethylcellulose, acrylate and methacrylate copolymers, water insoluble cellulosics, cellulose acetate, and cellulose acetate phthalate.
(17) The process according to any of the preceding items, wherein the aqueous latex or pseudolatex dispersion further comprises one or more components selected from the group consisting of colorants, plasticizer and emulsifier, wherein
   the colorant is selected from the group consisting of beta-carotene, indigo carmine, iron oxides, sunset yellow FCF, tartrazine, and titanium dioxide, or combinations thereof;
   the plasticizer is selected from the group consisting of phthalic derivatives, dibutylphthalate, butylphthalylbutylglycolate, propylene glycol, triacetine, silicone oil, diethylphthalate, triethyl citrate, dibutyl sebacate, polyethylene glycol, propylene glycol and combinations thereof; preferably the hydrophilic plasticizer is propylene glycol or triethyl citrate or combinations thereof, and more preferred the plasticizer is triethylcitrate;
   the emulsifier is selected from the group consisting of non ionic surfactants such as polysorbates, for instance polysorbate 80 or 20, or combinations thereof.
(18) The process according to any of the preceding items, wherein the aqueous latex or pseudolatex dispersion further comprises triethylcitrate, preferably in an amount of 1-7% by weight based on the aqueous latex or pseudolatex dispersion.
(19) The process according to any of the preceding items, wherein the amount of coating derived from the aqueous latex or pseudolatex dispersion as defined in items (16) to (18) is in the range of from 10 wt.-% to 40 wt.-%, further preferred in the range of from 10 wt.-% to 25 wt.-%, based on the total weight of the coated substrate.
(20) The process according to any of the preceding items, wherein the starting spray rate is from 8 g to 30 g of coating dispersion per m³ of process air per minute.
(21) The process according to any of the preceding items, wherein the spray pressure is from 0.5 bar to 8 bar, preferably from 1.0 bar to 5.0 bar.
(22) A production batch comprising coated substrates, wherein said substrates comprise acid-labile API and coalescent coating, wherein said coated substrates are obtained by applying a coating step using an aqueous latex or pseudolatex dispersion,
   and the coated substrates are essentially free from damages in the coating, and wherein the coalescent coating was not subjected to a heat treatment step comprising applying a temperature above a minimum film forming temperature of the coating.
(23) The production batch according to item (22), wherein at least 80%, preferably at least 85%, more preferably at least 90%, even more preferably at least 95% and most preferably at least 97% of the coated substrates of the production batch are free from visible damages.
(24) The production batch according to items (22) or (23), wherein essential freeness from damages is defined by an acid resistance test in which substrates directly coated by the coalescent coating are characterized by a limited release of the acid-labile API of equal to or less than 10% % within 2 hours in a test apparatus 2, 100 rpm, in 0.1 N hydrochloric acid, pH 1.1.
(25) The production batch according to any of items (22) to (24), wherein the limited release of the acid-labile API is equal to or less than 8%, preferably less than 7%.

In a further preferred embodiment, the limited release of the acid-labile API is equal to or less than 5%, or equal to or less than 2%.

In a preferred embodiment, the acid resistance test is the acid resistance test according to the USP protocol as described herein.

Within the meaning of the present invention, a "coalescent coating process" denotes a coating process by which latex particles come into contact with one another and unite to form a continuous, uniform and homogeneous film. Accordingly, the resulting coalescent coating is a coating that is essentially free from damages such as cracks and fissures. Furthermore, said coating has a uniform polymer distribution.

Within the meaning of the present invention, the term "damage" denotes any structural feature that leads to an undesired release of the API (drug) from the coated substrate/particle, and/or to an undesired dissolution profile of the API in a defined milieu. Damages can for instance be cracks, fissures or breakings. An undesired release of API from the coated substrate can for instance be determined by any suitable method that is known to a person skilled in the art, for instance by an acid resistance test as described elsewhere herein. A release of API is undesired if e.g. more than 10% of the API (drug) is released within 2 hours when subjected to the acid resistance test described herein. In other words, a coating is free from damages if, when applying the acid resistance test described herein, a limited amount of (acid-labile) API of e.g. equal to or less than 10% is released within 2 hours.
The excellent acid resistance is indicative for the improved structure and coalescence of the latex coating achieved by the present invention without heat treatment.

Whether the coated substrates are essentially or entirely free from visible damages can further (or additional) be determined by suitable imaging methods that are known to a person skilled in the art, such as by scanning electron microscopy (SEM).
(26) A production batch according to any of items (22) to (25), wherein the acid-labile API is selected form the group consisting of lansoprazole, omeprazole, esomeprazole, pantoprazole, rabeprazole, leminoprazole, hydroxyomeprazole, pariprazole, perprazole, tenatoprazole, and pharmaceutically acceptable salts thereof, preferably from the group consisting of lansoprazole, omeprazole, esomeprazole, pantoprazole, rabeprazole, and pharmaceutically acceptable salts thereof, and more preferably the acid-labile API is omeprazole or a pharmaceutically acceptable salt thereof.
(27) A production batch according to any of items (22) to (26), wherein the coating step comprises a coating phase carried out as defined in any of items (1) to (10).
(28) The production batch according to any of items (22) to (27), wherein the aqueous latex or pseudolatex dispersion is as defined in any of items (16) to (18).
(29) The production batch according to any of items (22) to (28), wherein the coalescent coating of the coated substrates has a mean thickness of about 40 µm or less, preferably of about 30 µm or less, and more preferably of about 20 µm or less.

In a preferred embodiment, in addition, the mean thickness of the coalescent coating is at least about 10 µm. In a further embodiment, the mean thickness of the coalescent coating is at least about 19 µm.
(30) The production batch according to any of items (22) to (29), wherein the coalescent coating of the coated substrates has a mean thickness of about 4.5% or less, preferably of about 4.0% or less, of the mean particle size (diameter) of the coated substrates.

In a further embodiment, the coalescent coating of the coated substrates has a mean thickness of about 3.5% or less, preferably of about 3.0% or less, of the mean particle size of the coated substrates.

In a further embodiment, the coalescent coating of the coated substrates has a mean thickness of about 2.5% or less of the mean particle size of the coated substrates.

In a further embodiment, the coalescent coating of the coated substrates preferably additionally has a mean thickness of at least about 1.0%, preferably of at least about 1.5% or about 2% of the mean particle size of the coated substrates.

With regard to the meaning of the terms and preferred embodiments of items (22) to (30), in particular with regard to the meaning of the terms "coalescent" and "coalescent coating", "substrate", "coating step", "coating phase" and "coating", reference is made to the specification elsewhere herein, as well as with regard to the advantages denoted elsewhere herein.
(31) A coated substrate comprising acid-labile API and coalescent coating, wherein said coating has a thickness of about 40 µm or less, preferably of about 30 µm or less, and more preferably of about 20 µm or less, and wherein said coated substrate is obtained by applying a coating step using an aqueous latex or pseudolatex dispersion.

In a preferred embodiment, in addition, the thickness of the coalescent coating is at least about 10 µm. In a further embodiment, the thickness of the coalescent coating is at least about 19 µm.
(32) The coated substrate according to item (31), wherein the thickness of the coalescent coating is in a range of from about 10 µm to about 40 µm, preferably of from about 10 µm to about 30 µm, more preferably of from about 10 µm to about 20 µm.
(33) A coated substrate comprising acid-labile API and coalescent coating, wherein the coalescent coating of the coated substrate has a thickness of about 4.5% or less, preferably of about 4.0% or less, of the particle size (diameter) of the coated substrate, and wherein said coated substrate is obtained by applying a coating step using an aqueous latex or pseudolatex dispersion.

In a further embodiment, the coalescent coating of the coated substrate has a thickness of about 3.5% or less, preferably of about 3.0% or less, of the particle size of the coated substrate.

In a further embodiment, the coalescent coating of the coated substrate has a thickness of about 2.5% or less of the particle size of the coated substrate.

In a further embodiment, the coalescent coating of the coated substrate preferably additionally has a thickness of at least about 1.0%, preferably of at least about 1.5% or about 2% of the particle size of the coated substrate.
(34) The coated substrate according to item (33), wherein the coalescent coating of the coated substrate has a thickness of from about 1.0% to about 4.0%, preferably of from about 1.0% to about 3.5%, of the particle size of the coated substrate.

With regard to the meaning of the terms and preferred embodiments of items (22) to (34), in particular with regard to the meaning of the terms "coalescent" and "coalescent coating", "substrate", "coating step", "coating phase" and "coating", reference is made to the specification elsewhere herein, as well as with regard to the advantages denoted elsewhere herein.

Further, in a preferred embodiment, the coalescent coating has not been subjected to a heat treatment step comprising applying a temperature above a minimum film forming temperature of the coating, and/or the coalescent coating is essentially free from damages such as cracks and fissures. Whether the coated substrate (or the coating, respectively) is (essentially) free from damages can be determined as described elsewhere herein. In particular, freeness from damages, while at the same time ensuring relatively low thickness of the coated substrate (or the coating, respectively), is defined by an acid resistance test in which the substrate directly coated by the coalescent coating is characterized by a limited release of the acid-labile API of equal to or less than 10% within 2 hours in a test apparatus 2, 100 rpm, in 0.1 N hydrochloric acid, pH 1.1.
(35) The coated substrate according to any of items (31) to (34), wherein the coating step comprises a coating phase carried out as defined in any of items (1) to (10).
(36) The coated substrate according to any of items (32) to (35), wherein the aqueous latex or pseudolatex dispersion is as defined in any of items (16) to (18).
(37) The coated substrate according to any of items (31) to (36), wherein the acid-labile API is as defined in item (26).

With regard to the meaning of the terms "coalescent" and "coalescent coating", "substrate", "coating step", "coating phase" and "coating", reference is made to the specification elsewhere herein, as well as with regard to the advantages denoted elsewhere herein.

### Detailed description of the invention

The present invention is now described in more detail by preferred embodiments and examples, which are however presented for illustrative purpose only and shall not be understood as limiting the scope of the present invention in any way.

In the process of coating a substrate, typically droplets of the coating dispersion fall on a solid substrate, water evaporates and the latex particles come closer to each other until they merge into a coating film. This step, or the process conditions applied during this step, respectively, has/have been found crucial for complete particle coalescence, and, thus, for providing a uniform film coating. In this process step, the coating conditions are very important with regard to the resulting coating. For instance, if aqueous latex or pseudolatex dispersions are used for coating a substrate, the resulting coating may suffer from disadvantages, such as crack formation e.g. caused by incomplete film formation of latex particles. This may have a negative impact on the dissolution properties of the dosage form due to film coating leakage and, thus, increased speed of drug release. In addition, the acid protection of an acid-sensitive API by the coating is reduced due to crack formation, since acid fluids may enter the core of the dosage form. Thus, it is important to have a process that provides a uniform film having coalescent properties.

Within the framework of the present invention it has now unexpectedly been found that the process of coating a substrate, e.g. a spray coating process, with an aqueous latex or pseudolatex dispersion under conditions as defined herein, provides for an improved process for the manufacture of a coated substrate. The process described herein can advantageously provide a coalescent coating without, if desired, the necessity of carrying out a heat treatment step comprising applying a temperature above a minimum film forming temperature of the coating. In other words, by applying the process according to the present invention, a coalescent coating can be provided onto a substrate, while at the same time, if desired, omitting a curing step. By omitting the curing step, the process can further be simplified and the processing time can be reduced further. Additionally, it does not require controlling the humidity during a curing step which also helps simplifying the process. Moreover, by omitting a heat treatment step, the risk of API degradation by heat treatment is avoided.

Furthermore, the process described herein is simplified in that it is not required to take any measures for increasing the humidity in the coating chamber by addition of water such as by adding water to the coating dispersion (reducing the solid content thereof), or adding water to the air that is introduced into the coating chamber (e.g. the inlet air). By setting the spray rate as described herein, a simple measure is provided for adjusting, preferably maintaining or increasing the relative air humidity inside the coating chamber at a certain level.

In typical coating devices, there is an outflow of "outlet air" from the coating chamber. In devices, e.g. fluid bed spray-coating devices, where the outlet air humidity essentially corresponds to the humidity inside the coating chamber, especially in devices where a high flow rate is applied, the relative air humidity in the outlet air from the coating chamber can be measured and thus determined instead of measuring and thus determining the relative air humidity inside the coating chamber.

Moreover, applying the process according to the present invention may contribute to preventing the tendency or occurrence of sticking together of the substrate to be coated, and/or of the coated substrate, during the coating step, in particular during the coating phase. This may further contribute to improving the quality of the obtained product, such as the coated tablet, as an enhanced proportion of the product batch may fulfil regulatory requirements such as measured by using the standard USP test for enteric coating.

The process parameters of the coating process, e.g. spray-drying or spray-coating process, such as solid content of the latex dispersion, product temperature, and defined relative air humidity in the coating chamber, as well as adjusting said defined relative air humidity at the desired level by setting a spray rate of the aqueous latex or pseudolatex dispersion as described herein, provide for the unexpected advantages described herein.

Therefore, the invention relates to a process for the manufacture of a coated substrate having coalescent coating, comprising the following steps:
a) providing an aqueous latex or pseudolatex dispersion with a solid content of from 20% to 40% by weight (w/w),
b) coating the substrate with the aqueous latex or pseudolatex dispersion of step a),
   wherein
   the step of coating the substrate comprises a coating phase that is carried out at a defined relative air humidity which is measured or determined inside the coating chamber or in the outlet air from the coating chamber, with said defined relative air humidity being at least 35% during a time period of at least 60%, preferably a time period of at least 70%, further preferred of at least 80% or 100%, of the duration of the coating phase and at a product temperature of at least 30°C,
   wherein the relative air humidity is adjusted by setting a spray rate of the aqueous latex or pseudolatex dispersion,
   and
c) obtaining a coated substrate having coalescent coating.

Within the meaning of the present invention, a "coalescent coating process" denotes a coating process by which latex particles come into contact with one another and unite to form a continuous, uniform and homogeneous film. Accordingly, the resulting coalescent coating is a coating that is essentially free from damages such as cracks and fissures. Furthermore, said coating has a uniform polymer distribution. Suitable methods for determining whether a coating is coalescent, i.e. uniform and being essentially free from damages such as cracks or fissures are known to a person skilled in the art. For instance, the acid resistance test (USP) can be applied. Preferably, the acid resistance test is carried out by using the USP protocol (United States Pharmacopeia 34) with the following conditions: *Medium:* 0.1 N hydrochloric acid; *Apparatus* 2: 100 rpm. *Time:* 2 hours. In short, the product is put into dissolution baskets and is sinked into thermostated 0.1 N HCl (pH 1.1). Baskets are rotated with 100 rpm. After 2h the pellets are transferred from HCl and assay of the drug is determined. The amount of drug dissolved in acid phase is calculated as 100% - assay of the drug determined after dissolution in 0.1 N HCl.

Within the meaning of the present invention, a coating is coalescent if equal to or less than 10%, preferably equal to or less than 8% or equal to or less than 7%, of the API (drug) is released within 2 hours when subjected to the above acid resistance test.

Additionally, the presence of a coalescent coating, or the presence or absence of damages in the coating such as cracks or fissures, can be determined by using SEM (scanning electron microscopy). Said measurement is carried out at a magnification at which cracks (if they occurred) per substrate particle can be seen and which is typically applied in this context, preferably of at least x150 magnification, such as x250 or x300 magnification (1.0keV) and picture resolution of 1280x1024 pixels. A coating is termed essentially free from damages, if at typically applied SEM conditions not more than 2, preferably not more than 1, and most preferably no crack is/are seen per substrate particle.

In the present invention, the coating phase is carried out at a defined relative air humidity of at least 35% during a time period of at least 60% of the duration of coating phase. In a typical process, the air humidity inside the coating chamber is increased at the beginning of the coating phase because water is introduced into the coating chamber due to spraying the aqueous latex or pseudolatex dispersion into said coating chamber. After some time, the air humidity inside the coating chamber reaches the desired level of at least 35% and is then adjusted as described herein.

Absolute humidity is usually measured in grams of water vapour per cubic meter of air volume.

The relative air humidity inside the coating chamber can be measured and thus determined by applying common measuring devices suitable for such purpose. These devices can be part of the coating apparatus used, or these devices can be placed to the coating apparatus.

Typically, in coating devices (such as fluid bed coaters, (perforated) pan coaters) the air flow is so high that the air temperature drop from the product to the exit of the coating chamber is very small, if there is any at all. Therefore, within the meaning of the present invention, measurement of relative air humidity in the outlet air exiting from the coating chamber can be equally taken as a measure for the same value of relative air humidity inside the coating chamber.

Preferably, the relative humidity is measured at the exit of the coating chamber. It is also possible to measure the humidity at locations spaced from the exit of the coating chamber. In this case, it is preferred to measure absolute humidity and then calculate the relative humidity inside the coating chamber. It is also possible to determine relative humidity inside the coating chamber by calculation based on the humidity (relative humidity, absolute humidity, or both) of inlet air, the amount of water (from coating dispersion) sprayed into the coating chamber and the applied (known) air flow.

A device that is used for carrying out the process according to the present invention can have a control system, monitoring various process parameters, such as the product temperature and the relative air humidity inside the coating chamber or in the outlet air. If one (or more) of said process parameters is/are outside a desired range, the device can (automatically or manually) adjust the process conditions in order that the process parameter/s brought back to the desired range as specified herein. In the present invention, the relative air humidity can be controlled/monitored and the spray rate of the coating dispersion can be adjusted or set to bring the relative air humidity to the desired range. The controlling and/or adjustment can be done automatically (e.g. by using suitable electronic devices) or manually.

In the present invention, the substrate to be coated and undergoing the coating process, has a temperature of equal to or more than 30°C during the coating phase. This temperature is commonly referred to as "product temperature". It is known to a person skilled in the art how the product temperature can be set to a desired level, e.g. in a typical coating device by using respective control means. Within the meaning of the present invention, any suitable method can be used for measuring the product temperature, if desired. In a preferred embodiment, the product temperature is measured by using a temperature probe which is positioned inside the coating chamber in such way that the probe is completely surrounded by product.

When adjusting the relative air humidity inside the coating chamber in the range as indicated herein by setting a spray rate of the aqueous latex or pseudolatex dispersion, there is no need to apply further or other measures that aim at increasing the humidity in the coating chamber, and hence according to a preferred embodiment, such further or other measures are avoided. Thus, it is preferred to avoid increasing the total amount of water that is introduced into the coating chamber per time by applying means other than varying the spray rate for spraying the aqueous latex or pseudolatex dispersion into the coating chamber. Examples of such measures of increasing the total amount of water added to the coating chamber, which are preferably omitted, are for instance the (further) provision or addition of additional amounts of water during the coating step to the water that is already present in the aqueous latex or pseudolatex dispersion (i.e. preferably omitting diluting the dispersion), and/or spraying water into the coating chamber (for example omitting to spray water via a nozzle into the coating chamber), and/or addition of water to the process (inlet) air.

The solid content of the aqueous latex or pseudolatex dispersion is calculated based on the total amount of all solid substances that are dissolved in the amount of water that is used. In a preferred embodiment, the solid content of a pseudolatex or latex coating dispersion is from 27% to 40% by weight, preferably from 27% to 35% by weight, and more preferably from 28% to 34% by weight, based on the total amount of all solid substances that are dissolved in the amount of water that is used.
In an even more preferred embodiment, the aqueous latex or pseudolatex dispersion has a solid content of about 32% by weight.

The aqueous latex or pseudolatex dispersion can be provided by any suitable methods that are known to a person skilled in the art, such as for example the methods described by the manufacturers of the polymers which can be used for preparing aqueous latex or pseudolatex dispersions. In particular, the dispersion may be stirred in water to provide homogeneous properties of the dispersion. The dispersions may then be used prior to settling of the solid substances. It is also possible to firstly disperse the polymer(s) in water and then add the additional solid substances to the polymer dispersion. One may avoid vigorous stirring of the dispersion to avoid air inclusion.

In a preferred embodiment, the product temperature is more than 30°C, but at most 40°C. In a further embodiment, the product temperature is about 33°C.

Additionally preferred, the relative air humidity inside the coating chamber during the coating phase is 40-50%, at most 90% or 95%.

According to the invention, the defined relative air humidity is at least 35% during a specified time period. It is also possible that the relative air humidity inside the coating chamber during said time period is more than 35%, preferably more than 37%, further preferred more than 39%, further preferred 41% or more, even further preferred 43% or more. A particular preferred range is 40-50%. In further embodiments, said defined relative air humidity inside the coating chamber is 50% or more, 60% or more, 70% or more, or 80% or more, and even 90% or more. The maximum relative air humidity inside the coating chamber during step b) is the relative air humidity where the substrate to be coated as well as the coated substrate just does not yet aggregate. This value can easily be determined by a person skilled in the art.

During the coating phase, the relative air humidity is measured, and thus determined, and, if necessary and depending on the measurement/determination result, the spray rate of the coating dispersion is adapted to provide the desired relative air humidity inside the coating chamber as specified herein. Thus, the relative air humidity inside the coating chamber is adjusted in the range indicated elsewhere herein by setting a spray rate of the aqueous latex or pseudolatex dispersion.
In a preferred embodiment, the relative air humidity inside the coating chamber is maintained essentially constant at a value of at least 35%, and/or is increased from a value of at least 35%.

This means that in one embodiment of the process according to the present invention, the relative air humidity inside the coating chamber has a certain value, with this value being at least 35%, and, preferably, being 45%, at maximum 95%, and this value is maintained essentially constant during a period of time as defined herein, e.g. of at least 60% of the coating phase. Further, it is also possible that during said period of time of the coating phase the relative air humidity inside the coating chamber, being at least 35%, is increased, preferably until the maximum relative air humidity of 95% is reached.

Within the meaning of the present invention, the term "maintained essentially constant" denotes that the relative air humidity inside the coating chamber does not deviate more than +/- 8%, preferably not more than +/- 5% or +/- 3%, more preferably not more than +/-2% or +/-1% from a certain value.

The relative air humidity described herein is maintained in the range as indicated herein by setting the spray rate, preferably increasing the spray rate, of the coating dispersion. Thus, the process is preferably carried out by monitoring the relative air humidity inside the coating chamber (by either measuring or determining humidity inside the coating chamber or in the outlet air from the coating chamber) and, if necessary, adjusting the relative air humidity inside the coating chamber by setting a spray rate of the aqueous latex or pseudolatex dispersion. Measuring, and thus determining, the relative air humidity inside the coating chamber can be carried out by using commercially available measuring devices such as devices available from Vaisala (US). If relative or absolute humidity of the inlet air is measured relative or absolute humidity inside the coating chamber can easily be calculated as the sum of relative or absolute inlet humidity and spraying rate. Preferably, relative or absolute humidity is measured continuously throughout the device operation.

Measuring and thus determining the relative air humidity inside the coating chamber can for example be carried out every few seconds (e.g. 1-3 seconds) during the coating phase (i.e. the spraying step), and, if need be, the spray rate can be set accordingly. The spray rate can be manually or automatically controlled to the defined humidity set point. It was unexpectedly found within the meaning of the present invention that adapting, preferably increasing, the spray rate and, thus, the amount of polymer per time, does not result in inhomogeneous film formation on the substrate but rather provides high quality films, e.g. with regard to the obtained uniformity and homogeneity of the coating.

Additionally preferred, adjusting the relative air humidity inside the coating chamber in step b) is not conducted by providing additional amounts of water to the water that is already present in the aqueous latex or pseudolatex dispersion (meaning that said dispersion is not diluted during the coating phase). Providing additional amounts of water to the aqueous latex or pseudolatex dispersion (diluting the dispersion), which is preferably not done according to the invention, has the effect that the solid content is reduced during the coating phase, i.e. during applying/spraying of the aqueous latex or pseudolatex dispersion on a substrate. Thus, it is preferred to keep the solid content of the aqueous latex or pseudolatex dispersion (which is supplied to the coating chamber) essentially constant (e.g. +/-2%) during the spraying process (i.e. during the coating phase).

Additionally preferred, the relative humidity of the air inside the coating chamber is not maintained by spraying water (other than that contained in the aqueous latex or pseudolatex dispersion) into the coating chamber, and/or by diluting the aqueous latex or pseudolatex dispersion during the coating phase, and/or by adding water to the process air (such as the inlet air), and/or by reducing the inlet air temperature, and/or by reducing the inlet air flow, and/or by modulating the product temperature.

Further preferred, said relative air humidity inside the coating chamber is maintained during at least 65%, 70% or 75% of the coating phase, more preferably during at least 80% or 85%, even more preferred during at least 90% or 95% of the coating phase. This ensures an even more improved coating.

Additionally preferred, the absolute humidity of the outlet air is in a range of from 6.5 g/m³ to 25 g/m³, preferably in a range of from 8.0 g/m³ to 20 g/m³, further preferred in a range of from 9.0 g/m³ to 15 g/m³ or in a rage of from 10.0 g/m³ to 15 g/m³, preferably at an outlet air temperature in a range of from about 25°C to 40°C. Typically, the outlet air temperature (at the exit of the coating chamber) is equal to the product temperature. If the outlet air temperature is not measured directly at the exit of the coating chamber, the measured temperature will be lower and said temperature has to be corrected accordingly.

Typically and preferably, the inlet air temperature is higher than the outlet air temperature. During passage through the coating chamber, the introduced air takes up water and the energy required for that cools down the air to the resulting final outlet air temperature. Preferably, the relative outlet air humidity essentially corresponds to the humidity in the coating chamber during the coating phase and preferably, the relative outlet air humidity is measured and thus determined instead of the relative air humidity inside the coating chamber. In this case, the term "essentially" denotes that the outlet air humidity can exhibit deviations of e.g. up to +/- 5%, up to +/- 4%, up to +/- 3% or up to +/- 2% from the humidity in the coating chamber during the coating phase.

Adjusting the relative air humidity and product temperature allows adjusting the water outtake and thus the conditions for drying the aqueous latex or pseudolatex dispersion on the substrate.

Additionally or further preferred, the inlet air temperature applied is at least 40°C. In a further preferred embodiment, the inlet air temperature is at least 70°C. The inlet air temperature is chosen depending on the scale of production, wherein higher temperatures are used for bigger production scales.

Preferably, the absolute humidity of the inlet air is lower than the absolute humidity of the outlet air.

The product temperature, preferably the product bed temperature, during the spray coating process is equal to or more than 30°C, but preferably at most 40 or 45°C. After the coating phase has ended, a drying phase and/or cool-down phase may be applied, wherein the flow of inlet air can be reduced by 30-50% and/or the inlet air temperature can be reduced by 30-50% during said phase(s). The drying phase can for example last from 5 minutes to 1 hour, depending on the active ingredient moisture sensibility.

In a further embodiment, the maximum product temperature, preferably the product bed temperature, is about or preferably below the minimum film forming temperature of the respective coating. Within the meaning of the present invention, the term "film forming temperature" denotes the lowest temperature at which a latex or pseudolatex dispersion will uniformly coalesce when laid on a substrate e.g. as a thin film. Said film forming temperature can be determined for each latex or pseudolatex dispersion by any suitable method that is known to a person skilled in the art. In a preferred embodiment, the maximum product temperature, preferably the product bed temperature, is below 40°C or below 35°C.

In a further embodiment, the relative inlet air humidity is between 0% and 50%, preferably between 5% and 20%.

In a further preferred embodiment, the step of coating the substrate in step b) is carried out by applying said aqueous latex or pseudolatex dispersion onto the substrate by using a spraying process.

In a further embodiment, after step c) of the process described herein, a further coating is applied onto the coated substrate. Said further coating can include colorants and/or flavours. Depending on the intended use of the coated substrate, and/or on the type of coating that was applied to the substrate, a person skilled in the art can readily choose suitable colorant/s and/or flavour/s.

In a further embodiment, it is also possible that, prior to the coating of the substrate with the coating dispersion as described herein, one or more (sub-) coatings are provided on the substrate. Such sub-coating/s is/are for instance intermediate coating/s that e.g. protect the API that is present in the substrate form the coating resulting from the coating dispersion described herein.

The substrate which can be used for coating with aqueous latex or pseudolatex dispersion can be chosen by a person skilled in the art depending on the application of the coated substrate. Preferably, the substrate is selected from the group consisting of pellets, tablets, granules, grains, soft capsules, hard capsules, powders, beads, films, or spheroids. As described above, the substrate can comprise one or more (sub-) coatings. This/these (sub-) coating/s is/are present on the substrate below the coating that results from the coating dispersion described herein. Consequently, the term "substrate" also denotes substrates that do comprise one or more coating/s that are present below the coating that results from the coating dispersion described herein.

It is further possible, and it is preferred, that the substrate comprises one or more API. If the substrate comprises (sub-) coating/s, the API can (additionally) be comprised in this/these (sub-) coating/s, or only in this/these (sub-) coating/s. It is however possible that there is no API present in the (sub-) coating/s.

In general, when applying the process according to the present invention, any API, preferably any API where controlled release is desired, can be used. In accordance with the present invention, the term "controlled release" encompasses all kinds of controlled release, including slow release, sustained and delayed release, of the API.

In a preferred embodiment of the present invention, at least one acid-labile API is used. Within the meaning of the invention, the term "acid-labile" refers to compounds (or APIs, respectively) that comprise functional groups that are susceptible to the presence of protons. Due to these acid-labile functional groups, the compounds (or APIs) degrade at a pH of 5 or less to a substantial degree. The acid-labile APIs can for example be selected from the group consisting of pravastatin, fluvastatin, atorvastatin, penicillin G, ampicillin, streptomycin, clarithromycin, azithromycin, dideoxyinosine, dideoxyadenosine, dideoxycytosine, digoxin, pancreatin, bupropion, lansoprazole, omeprazole, esomeprazole, pantoprazole, rabeprazole, and leminoprazole, hydroxyomeprazole, pariprazole, perprazole, tenatoprazole, and pharmaceutically acceptable salts thereof. Further preferred, the acid-labile API is a prazole, preferably a prazole selected from the group consisting of lansoprazole, omeprazole, esomeprazole, pantoprazole, rabeprazole, and leminoprazole, hydroxyomeprazole, pariprazole, perprazole, tenatoprazole, and pharmaceutically acceptable salts thereof.

The coating material used in the process according to the present invention is an aqueous latex or pseudolatex dispersion. The term "aqueous latex or pseudolatex dispersion" refers to a dispersion of water insoluble film forming materials which during removal of water are capable of coalescing to form a coating on a substrate. Pseudolatexes can be prepared by emulsifying preformed polymers. This can e.g. be done by dissolving a polymer, e.g. ethylcellulose, in a suitable organic solvent, combining said solution with water to provide an emulsion, whereupon removal of the organic solvent by distillation a pseudolatex dispersion in water is obtained. When preparing pseudolatex or latex dispersions, it may be useful to use emulsifiers.

Preferably, the aqueous latex or pseudolatex dispersion comprises one or more polymers selected from the group consisting of ethylcellulose, acrylate and methacrylate copolymers, water insoluble cellulosics, cellulose acetate, and cellulose acetate phthalate. Preferred polymers, or polymer dispersions, respectively, are members of the Eudragit ® aqueous coating dispersion series of Evonik Industries. One particularly preferred polymer is sold under the tradename Eudragit® L30 D55 (Evonik industries, Germany).

In a further embodiment, the aqueous latex or pseudolatex dispersion further comprises one or more components selected from the group consisting of colorants, plasticizers and emulsifiers. In a preferred embodiment, the aqueous latex or pseudolatex dispersion comprises one or more components selected from the group of plasticizers as defined herein, and, optionally, one or more components selected from the group consisting of colorants and emulsifiers as defined herein.

Within the meaning of the present invention, any suitable colorant/s that is/are known to a person skilled in the art can be used. In a preferred embodiment, the colorant(s) are selected from the group consisting of beta-carotene, indigo carmine, iron oxides, sunset yellow FCF, tartrazine, and titanium dioxide, or combinations thereof.

Within the meaning of the present invention, any suitable plasticizer/s that is/are known to a person skilled in the art can be used. In a preferred embodiment, the plasticizer/s are selected from the group consisting of phthalic derivatives, dibutylphthalate, butylphthalylbutylglycolate, propylene glycol, triacetine, silicone oil, diethylphthalate, triethyl citrate, dibutyl sebacate, polyethylene glycol, propylene glycol and combinations thereof; preferably the hydrophilic plasticizer is propylene glycol and/or triethyl citrate, and more preferred the plasticizer is triethylcitrate, optionally in combination with one or more further colorants, plasticizers and emulsifiers as mentioned elsewhere herein.

Within the meaning of the present invention, any suitable emulsifier/s that is/are known to a person skilled in the art can be used. In a preferred embodiment, the emulsifier/s are selected from the group consisting of non ionic surfactants such as polysorbates, for instance polysorbate 80 or 20, or combinations thereof.

In one embodiment, the aqueous latex or pseudolatex dispersion comprises (in addition to the polymer/s as defined elsewhere herein) triethyl citrate and optionally one or more further components selected from the group consisting of colorant/s, plasticizer/s and emulsifier/s. In one embodiment, the aqueous latex or pseudolatex dispersion (in addition to the polymer/s as defined elsewhere herein) comprises triethyl citrate and one or more components selected from the group consisting of colorants, and emulsifiers, or comprises only triethyl citrate in addition to the polymer/s as defined elsewhere herein.

In addition to the polymer/s, colorant/s, plasticizer/s and/or emulsifier/s, the aqueous latex or pseudolatex dispersion may eventually further comprise one or more permeation enhancing agents which can increase the release rate of APIs. Such permeation enhancing agents can e.g. be carrageenan, alginates, starches, polymers (which themselves do not form latex or pseudolatex dispersions) or sugars.

In a further preferred embodiment, the aqueous latex or pseudolatex dispersion comprises the plasticizer/s, preferably triethyl citrate, in an amount of 1-7% by weight based on the aqueous latex or pseudolatex dispersion. It is also possible to use an amount of plasticizer/s, preferably triethyl citrate, of between 85:10 to 95:10 (latex or pseudolatex polymer/s:plasticizer/s). Preferably, the ratio of triethyl citrate to the polymer being present in the aqueous latex or pseudolatex dispersion is about 90:10.

Further preferred, the amount of coating resulting from the coating with the aqueous latex or pseudolatex dispersion described herein is between 10 wt.-% and 40 wt.-%, even further preferred between 10 wt.-% and 25 wt.-%, based on the total weight of the coated substrate. Preferably, the thickness of the coating provided by the process described herein is in a range of from 30 to 50 µm.

In the method according to the present invention, a preferred starting spray rate is in a range of from 8 to 30 g of dispersion per m³ of process air per minute. Additionally preferred, the process described herein is carried out in a batch-wise process with an amount of substrate per batch of at least 50 kg, preferably at least 100 kg, further preferred at least 150 kg with a maximum of e.g. 500 kg.

In the spray processes described herein it is preferred to introduce the aqueous latex or pseudolatex dispersion and the inlet air via separate nozzles. The spray rate of the aqueous latex or pseudolatex dispersion is adjusted in order to provide the desired relative air humidity.

Additionally preferred, the spray pressure is from 0.5 bar to 8 bar, preferably from 1.0 bar to 5.0 bar.

For spray-drying processes, for example the device "Huettlin HKC 200/200 disc jet" can be used. However, it is also possible to use other suitable devices. Suitable devices are known to a person skilled in the art, e.g. Glatt GPCG with wurster insert.

The present invention further refers to a production batch comprising coated substrates, wherein said substrates comprise acid-labile API and coalescent coating, wherein said coated substrates are obtained by applying a coating step using an aqueous latex or pseudolatex dispersion, and wherein at least 80% of the coated substrates are free from damages in the coating, and wherein the coalescent coating was not subjected to a heat treatment step comprising applying a temperature above a minimum film forming temperature of the coating.

Within the context of the present invention, water content of the final product can be measured as the loss on drying for instance by using the Karl-Fischer titration method.

Within the meaning of the present invention, the term "production batch" refers to the amount of coated substrates that are produced in groups, or batches, respectively, and not in a continuous stream.

With regard to the meaning of the terms of the production batch, in particular with regard to the meaning of the terms "coalescent" and "coalescent coating", "substrate", "coating step", "coating phase" and "coating", reference is made to the specification elsewhere herein, as well as with regard to the advantages denoted elsewhere herein. All process conditions and preferred embodiments described herein in respect to the process for the manufacture of a coated substrate can be applied when preparing the production batch according to the invention.

Within the meaning of the present invention, the term "damage" denotes any structural feature that leads to an undesired release of the API from the coated particle, and/or to an undesired dissolution profile of the API in a defined milieu. Damages can for instance be cracks, fissures or breakings. The release of the API from the coated substrate, as well as the dissolution profile of the API, can be determined by any suitable method that is known to a person skilled in the art, for instance an acid resistance test as described elsewhere herein. Whether the coated substrates are essentially or entirely free from visible damages can be determined by any suitable method that is known to a person skilled in the art, such as by scanning electron microscopy (SEM).

Additionally preferred, the acid-labile API is selected form the group consisting of lansoprazole, omeprazole, esomeprazole, pantoprazole, rabeprazole, leminoprazole, hydroxyomeprazole, pariprazole, perprazole, tenatoprazole, and pharmaceutically acceptable salts thereof, preferably from the group consisting of lansoprazole, omeprazole, esomeprazole, pantoprazole, rabeprazole, and pharmaceutically acceptable salts thereof, and more preferably the acid-labile API is omeprazole or a pharmaceutically acceptable salt thereof.

In a preferred embodiment, at least 85%, more preferably at least 90%, even more preferably at least 95% and most preferably at least 97% of the coated substrates of the production batch are free from damages.

Additionally preferred, the coating step comprises a coating phase that is carried out as defined elsewhere herein.

Additionally or further preferred, the aqueous latex or pseudolatex dispersion that is used for preparing the production batch is as defined elsewhere in the specification.

Furthermore, the invention refers to a coated substrate obtained or obtainable according to a process described herein.

The present invention also refers to the production batch according to the present invention, wherein the coalescent coating of the coated substrates has a mean thickness of about 40 µm or less, preferably of about 30 µm or less, and more preferably of about 20 µm or less. The thickness of the coating can for instance be measured by using optical methods such as SEM.

In a preferred embodiment, in addition, the mean thickness of the coalescent coating is at least about 10 µm. In a further embodiment, the mean thickness of the coalescent coating is at least about 19 µm.

In a further embodiment according to the present invention, the coalescent coating of the coated substrates of the production batch has a mean thickness of about 4.5% or less, preferably of about 4.0% or less, of the mean particle size (diameter) of the coated substrates.

In a further embodiment, the coalescent coating of the coated substrates of the production batch has a mean thickness of about 3.5% or less, preferably of about 3.0% or less, of the mean particle size of the coated substrates.

In a further embodiment, the coalescent coating of the coated substrates of the production batch has a mean thickness of about 2.5% or less of the mean particle size of the coated substrates.

In a further embodiment, the coalescent coating of the coated substrates of the production batch preferably additionally has a mean thickness of at least about 1.0%, preferably of at least about 1.5% or about 2% of the mean particle size of the coated substrates.

In a further embodiment, the coalescent coating of the coated substrates of the production batch has a mean thickness of from about 1.0% to about 4.0%, preferably of from about 1.0% to about 3.5%, of the mean particle size of the coated substrates.

With regard to the meaning of the terms and preferred embodiments of the production batch of the present invention, in particular with regard to the meaning of the terms "coalescent" and "coalescent coating", "substrate", "coating step", "coating phase" and "coating", reference is made to the specification elsewhere herein, as well as with regard to the advantages denoted elsewhere herein.

The present invention further refers to a coated substrate comprising acid-labile API, preferably the acid-labile API as defined elsewhere herein, and coalescent coating, wherein said coating has a thickness of about 40 µm or less, preferably of about 30 µm or less, and more preferably of about 20 µm or less, and wherein said coated substrate is obtained by applying a coating process using an aqueous latex or pseudolatex dispersion, wherein preferably the coalescent coating is essentially free from damages, with essential freeness from damages being defined by an acid resistance test in which the substrate directly coated by the coalescent coating is characterized by a limited release of the acid-labile API of equal to or less than 10% within 2 hours in a test apparatus 2, 100 rpm, in 0.1 N hydrochloric acid, pH 1.1.

In a preferred embodiment, in addition, the thickness of the coalescent coating is at least about 10 µm. In a further embodiment, the thickness of the coalescent coating is at least about 19 µm.

In a further embodiment, the thickness of the coalescent coating of the coated substrate is in a range of from about 10 µm to about 40 µm, preferably of from about 10 µm to about 30 µm, more preferably of from about 10 µm to about 20 µm.

The present invention further refers to a coated substrate comprising acid-labile API, preferably the acid-labile API as defined elsewhere herein, and coalescent coating, wherein the coalescent coating of the coated substrate has a thickness of about 4.5% or less, preferably of about 4.0% or less, of the particle size (diameter) of the coated substrate, and wherein said coated substrate is obtained by applying a coating step using an aqueous latex or pseudolatex dispersion, wherein preferably the coalescent coating is free from damages, with freeness from damages being defined by an acid resistance test in which the substrate directly coated by the coalescent coating is characterized by a limited release of the acid-labile API of equal to or less than 10% within 2 hours in a test apparatus 2, 100 rpm, in 0.1 N hydrochloric acid, pH 1.1.

In a further embodiment, the coalescent coating of the coated substrate has a thickness of about 3.5% or less, preferably of about 3.0% or less, of the particle size of the coated substrate.

Again, with regard to the meaning of the terms and preferred embodiments of the coated substrate of the present invention, in particular with regard to the meaning of the terms "coalescent" and "coalescent coating", "substrate", "coating step", "coating phase" and "coating", reference is made to the specification elsewhere herein, as well as with regard to the advantages denoted elsewhere herein.

Further, in a preferred embodiment, the coalescent coating of the coated substrate according to the present invention as well as the coalescent coating of the coated substrates of the production batch of the present invention has/have not been subjected to a heat treatment step comprising applying a temperature above a minimum film forming temperature of the coating.

In a preferred embodiment, the coating step comprises a coating phase carried out as defined elsewhere herein, and/or the aqueous latex or pseudolatex dispersion is as defined elsewhere herein.

With regard to the meaning of the terms "coalescent" and "coalescent coating", "substrate", "coating step", "coating phase" and "coating", reference is made to the specification elsewhere herein, as well as with regard to the advantages denoted elsewhere herein.

### Methods

### SEM (Scanning electron microscopy) pictures

SEM pictures were taken with:
JEOL JSM-7001 FE-SEM device; Samples were dusted with Au 5µm prior measurement. Measurements were taken at 1 kV or 1.5 kV, SEI SEM.

### Acid resistance test (USP)/Testing coalescence of a coat

Acid resistance test according to USP (also mentioned above): *Medium:* 0.1 N hydrochloric acid; *Apparatus* 2: 100 rpm. *Time:* 2 hours.

### Determining the (mean) coating thickness

The thickness of the coating can for instance be determined by SEM.

The mean thickness of the coating of a plurality of coatings, as e.g. present in production batches, can for instance be measured by measuring the thickness of the coating by using SEM of 100 coated substrates, and taking the average of said measurements.

### (Mean) particle size

The mean particle size can be measured by any suitable standard method that is known to a person skilled in the art, for instance by Laser Diffraction Methods, optical counting methods, sieve methods, by photoanalytical methods, or by microscopic methods. Preferably, the mean particle size is measured by microscopic methods by measuring the particle size of a suitable amount of coated substrates (e.g. 100 coated substrates), and taking the average of said measurements. If the mean particle size of the coated substrates is expected to be in the µm-range, an SEM is used.

The particle size of a single coated substrate can also be measured by any suitable method that is known to a person skilled in the art, e.g. by the methods above. Preferably, the particle size of a single coated substrate is measured by microscopic methods. If the particle size of the coated substrate is expected to be in the µm-range, an SEM is used.

### Description of the figures

The pictures in Fig. 1, 2 and 3 were scaled down to approximately 290x220 pixels from 1280x1024 pixels.

### Fig. 1: SEM pictures of pellets having a uniform, coalescent film

This figure shows a coated pellet (coated substrate) that has been prepared according to the inventive method. The coating covering the pellet (substrate) is coalescent and uniform. Figure A) shows the coated pellet with 50x magnification, figure B) shows the coated pellet with 300x magnification.

### Fig. 2: SEM pictures of pellets having a uniform, coalescent film

This figure shows a coated pellet that has been prepared according to the inventive method. The coating covering the pellet is coalescent and uniform. Figure A) shows the coated pellet with 50x magnification, figure B) shows the coated pellet with 250x magnification.

### Fig. 3: SEM pictures of pellets showing a film having fissures and breakings

This figure shows a coated pellet that has been prepared by using a comparative coating method. Figure A) shows the pellet with 75x magnification, figure B) shows the coated pellet with 350x magnification.

### Examples

### Example 1:

### Pellet (coated substrate) composition:

| Substance | Amount per tablet core (mg) |
|---|---|
| Pellet core | 210,000 |
| Eudragit L30 D55 | 105,000 (31,500 dry substance) |
| Triethyl citrate | 3,500 |
| Total mass (mg) | 245,000 |

### Manufacturing procedure:

168 kg of pellet cores (substrate) were loaded into fluid bed coating device (Huettlin HKC 200/200 disc jet) equipped with 12 spray nozzles.

| Parameter | Set point |
|---|---|
| Air flow [m³/h] | 3500 |
| Inlet air temperature [°C] | 71 |
| Inlet air humidity [g/kg] | 1 |
| Spray rate [g/min] | 1200 |
| Spray pressure [bar] | 3.0 |
| Observed values | |
| Product temperature [°C] | 32 |
| Outlet air temperature [°C] | 33 |
| Calculated values | |
| Outlet air humidity - absolute [g/m³] | 14.7 |
| Outlet air humidity - relative [%] | 43.4 |

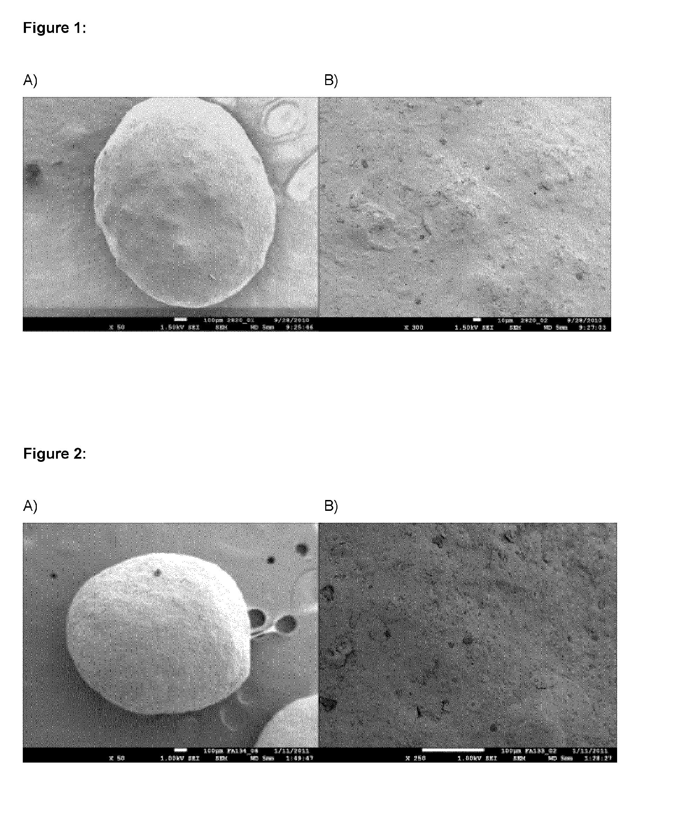

A coated pellet (coated substrate) obtained by said method is shown in Fig. 1. No damages (e.g. fissures or breakings) are present.

### Acid resistance test (USP):

Amount of drug released in 2h (acid phase): 1.3%

### Example 2:

### Pellet (coated substrate) composition:

| Substance | Amount per tablet core (mg) |
|---|---|
| Pellet core | 210,000 |
| Eudragit L30 D55 | 120,000 (36,000 dry substance) |
| Triethyl citrate | 4,000 |
| Total mass (mg) | 250,000 |

### Manufacturing procedure:

484 g of pellet cores (substrate) were loaded into fluid bed coating device (Huettlin HKC 5 disc jet) equipped with two spray nozzles.

| Parameter | Set point |
|---|---|
| Air flow [m³/h] | 170 |
| Inlet air temperature [°C] | 42 |
| Inlet air humidity [g/kg] | 2 |
| Spray rate [g/min] | 30 |
| Spray pressure [bar] | 0.7 |
| Observed values | |
| Product temperature [°C] | 30 |
| Outlet air temperature [°C] | 33 |
| Calculated values | |
| Outlet air humidity - absolute [g/m³] | 10.8 |
| Outlet air humidity - relative [%] | 40.6 |

A coated pellet (coated substrate) obtained by said method is shown in Fig. 2. No damages (e.g. fissures or breakings) are present.

### Acid resistance test (USP):

Amount of drug released in 2h (acid phase): 6.1 %.

### Example 3

### COMPERATIVE EXAMPLE 1

### Pellet (coated substrate) composition:

| Substance | Amount per tablet core (mg) |
|---|---|
| Pellet core | 210,000 |
| Eudragit L30 D55 | 105,000 (31,500 dry substance) |
| Triethyl citrate | 3,500 |
| Total mass (mg) | 250,000 |

### Manufacturing procedure:

484 g of pellet cores (substrate) were loaded into fluid bed coating device (Huettlin HKC 5 disc jet) equipped with two spray nozzles.

| Parameter | Set point |
|---|---|
| Air flow [m³/h] | 170 |
| Inlet air temperature [°C] | 42 |
| Inlet air humidity [g/kg] | 2 |
| Spray rate [g/min] | 15.1 |
| Spray pressure [bar] | 0,6 |
| Observed values | |
| Product temperature [°C] | 30 |
| Outlet air temperature [°C] | 33 |
| Calculated values | |
| Outlet air humidity - absolute [g/m³] | 6.5 |
| Outlet air humidity - relative [%] | 24.6 |

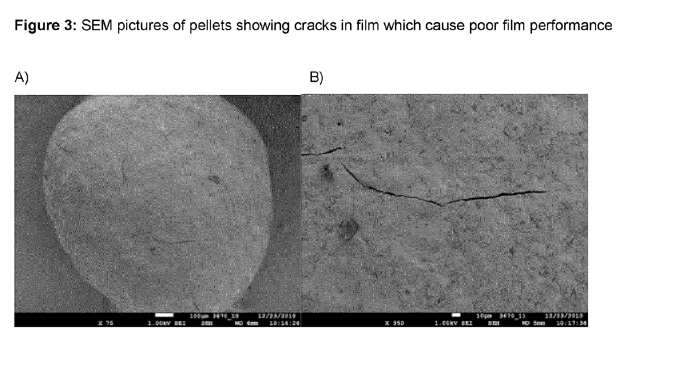

A coated pellet (coated substrate) obtained by the above comparative method is shown in Fig. 3. Several damages are seen.

### Acid resistance test (USP):

Amount of drug released in 2h (acid phase): 19.6%

## Claims

1. A process for the manufacture of a coated substrate having a coalescent coating, the process comprising the following steps:
a) providing an aqueous latex or pseudolatex dispersion with a solid content of from 20% to 40% by weight,
b) coating the substrate by spraying with the aqueous latex or pseudolatex dispersion of step a), wherein
the step of coating the substrate comprises a coating phase that is carried out at a defined relative air humidity which is measured or determined inside the coating chamber or in the outlet air from the coating chamber, with said defined relative air humidity being at least 35% during a time period of at least 60% of the duration of the coating phase, and at a product temperature of at least 30°C,
wherein the relative air humidity is adjusted by setting a spray rate of the aqueous latex or pseudolatex dispersion,
and
c) obtaining a coated substrate having coalescent coating.

2. The process according to claim 1, wherein during said at least 60% of the duration of the coating phase the relative air humidity inside the coating chamber is maintained essentially constant at a value of at least 35%, and/or is increased from a value of at least 35%.

3. The process according to claim 1 or 2, wherein maintaining the relative air humidity essentially constant, and/or increasing the relative air humidity, is performed by increasing the spray rate of the aqueous latex or pseudolatex dispersion.

4. The process according to any of the preceding claims, wherein after step b) no heat treatment step comprising applying a temperature above a minimum film forming temperature of the coating is carried out.

5. The process according to any of the preceding claims, wherein in step b) the relative air humidity is maintained essentially constant, or increased, without providing further amounts of water in addition to the water that is already present in the aqueous latex or pseudolatex dispersion, and/or without spraying water into the coating chamber, and/or without addition of water to the process air.

6. The process according to any of the preceding claims, wherein the substrate comprises pharmaceutically active ingredient (API), wherein said API preferably is acid-labile API, wherein more preferably the acid-labile API is selected from the group consisting of lansoprazole, omeprazole, esomeprazole, pantoprazole, rabeprazole, leminoprazole, hydroxyomeprazole, pariprazole, perprazole, tenatoprazole, and pharmaceutically acceptable salts thereof, even more preferably from the group consisting of lansoprazole, omeprazole, esomeprazole, pantoprazole, rabeprazole, or pharmaceutically acceptable salts thereof, and most preferably the acid-labile API is omeprazole or a pharmaceutically acceptable salt thereof.

7. The process according to any of the preceding claims, wherein the aqueous latex or pseudolatex dispersion comprises one or more polymers selected from the group consisting of ethylcellulose, acrylate and methacrylate copolymers, water insoluble cellulosics, cellulose acetate, and cellulose acetate phthalate.

8. The process according to any of the preceding claims, wherein the aqueous latex or pseudolatex dispersion further comprises triethylcitrate, preferably in an amount of 1-7% by weight based on the aqueous latex or pseudolatex dispersion.

9. The process according to any of the preceding claims, wherein the amount of coating derived from the aqueous latex or pseudolatex dispersion is in the range of from 10 wt.-% to 40 wt.-%, further preferred in the range of from 10 wt.-% to 25 wt.-%, based on the total weight of the coated substrate.

10. The process according to any of the preceding claims, wherein the starting spray rate is from 8 g to 30 g of dispersion per m³ of process air per minute.

11. The process according to any of the preceding claims, wherein the spray pressure is from 0.5 bar to 8 bar, preferably from 1.0 bar to 5.0 bar.

12. A production batch comprising coated substrates, wherein said substrates comprise acid-labile API and coalescent coating, wherein said coated substrates are obtained by applying a coating step using an aqueous latex or pseudolatex dispersion,
and the coated substrates are essentially free from damages in the coating, and
wherein the coalescent coating was not subjected to a heat treatment step comprising applying a temperature above a minimum film forming temperature of the coating, wherein essential freeness from damages is defined by an acid resistance test in which substrates directly coated by the coalescent coating are **characterized by** a limited release of the acid-labile API of equal to or less than 10% within 2 hours in a test apparatus 2, 100 rpm, in 0.1 N hydrochloric acid, pH 1.1.

13. A coated substrate comprising acid-labile API and coalescent coating, wherein said coating has a thickness of about 40 µm or less, and wherein said coated substrate is obtained by applying a coating process using an aqueous latex or pseudolatex dispersion, and wherein said coalescent coating is essentially free from damages, with essential freeness from damages being defined by an acid resistance test in which the substrate directly coated by the coalescent coating is **characterized by** a limited release of the acid-labile API of equal to or less than 10% within 2 hours in a test apparatus 2, 100 rpm, in 0.1 N hydrochloric acid, pH 1.1.

14. A production batch according to claim 12, or a coated substrate according to claim 13, wherein the acid-labile API is selected form the group consisting of lansoprazole, omeprazole, esomeprazole, pantoprazole, rabeprazole, leminoprazole, hydroxyomeprazole, pariprazole, perprazole, tenatoprazole, and pharmaceutically acceptable salts thereof, preferably from the group consisting of lansoprazole, omeprazole, esomeprazole, pantoprazole, rabeprazole, and pharmaceutically acceptable salts thereof, and more preferably the acid-labile API is omeprazole or a pharmaceutically acceptable salt thereof.

15. A production batch according to claim 12 or 14, or a coated substrate according to claim 13 or 14, wherein the coating step comprises a coating phase carried out as defined in any of claims 1 to 5.
